# FASCICULE DE BREVET EUROPEEN

(11) **EP 0 898 960 B1**
(45) Date de publication et mention de la délivrance du brevet: **25.07.2001**
(21) Numéro de dépôt: 98401988.5
(22) Date de dépôt: 04.08.1998
(51) Int. Cl.: A61K 7/48

(54) **Composition épaissie comprenant de la silice pyrogénée**
Verdikte Zusammensetzung enthaltend pyrogene Siliciumdioxid
Thickened composition containing pyrogenic silica

(30) Priorité: 28.08.1997 FR 9710758
(43) Date de publication de la demande: 03.03.1999
(73) Titulaire: L'OREAL, 75008 Paris (FR)
(72) Inventeur: Arnaud, Pascal, 94340 L'Hay-Les-Roses (FR)
(74) Mandataire: Lhoste, Catherine

(56) Documents cités:
- EP-A- 0 708 114
- EP-A- 0 776 654
- MAJEWICZ ET AL.: "oil-based cosmetic and therapeutic compositions containing ethylguar" RESEARCH DISCLOSURE, no. 37807, octobre 1995, page 642 XP002067837

## Description

L'invention se rapporte à une composition épaissie contenant de la silice pyrogénée associée à un nouvel épaississant, destinée en particulier aux domaines cosmétique et/ou dermatologique. L'invention se rapporte aussi à une utilisation de cette composition pour le traitement des matières kératiniques telles que la peau, les ongles, les cils, les sourcils, les cheveux ou des muqueuses telles que les lèvres et l'intérieur des paupières.

Dans les compositions cosmétiques et dermatologiques, il est courant d'épaissir des phases non aqueuses pour obtenir la consistance souhaitée. Les compositions épaissies permettent de faciliter la prise du produit hors de son conditionnement sans perte significative, de limiter la diffusion du produit à la zone locale de traitement, de répartir le produit de façon régulière sur la zone locale de traitement ou bien encore de pouvoir utiliser le produit dans des quantités suffisantes pour obtenir l'effet cosmétique ou dermatologique recherché.

Cet épaississement est primordial pour les compositions telles que celles des produits pour le soin, l'hygiène ou le maquillage comme les rouges à lèvres qui doivent bien se répartir de façon homogène sur la surface locale à traiter ainsi que pour les compositions capillaires qui doivent s'étaler et se répartir de façon régulière le long des fibres kératiniques et ne pas ruisseler sur le front, la nuque, le visage ou dans les yeux.

Pour épaissir les compositions, il est connu d'ajouter de la silice pyrogénée comme agent épaississant (voir la demande FR-A-1453089). Lorsque l'on veut préparer des compositions suffisamment épaissies, il est nécessaire d'ajouter une quantité importante de silice pyrogénée. Ceci est notamment le cas lorsque l'on souhaite épaissir une composition comprenant un milieu polaire comme par exemple l'acétate d'éthyle, l'éthanol ou les huiles d'origine végétale. Or, l'addition d'une quantité importante de cet épaississant altère les propriétés cosmétiques de la composition : en effet, la composition devient moins confortable lors de l'application et au cours du temps après application. Ceci est notamment le cas pour un rouge à lèvres où le film de rouge est peu confortable à porter sur les lèvres et provoque une sensation de tiraillement. En outre, la composition a tendance à dessécher les lèvres et à présenter une couvrance peu satisfaisante.

Il est également connu d'associer à la silice pyrogénée un autre agent épaississant. Par exemple, la demande EP-A-776654 décrit une composition cosmétique comprenant de la silice amorphe et de la gomme de xanthane ; le brevet US-A-5071639 décrit une composition de vernis à ongles comprenant de la silice hydrophile ou hydrophobe et une argile comme la bentone. Or les argiles ont l'inconvénient d'opacifier et de matifier la composition et il n'est pas possible d'obtenir des compositions transparentes ou translucides d'aspect brillant.

Le but de la présente invention est de proposer une composition transparente ou translucide épaissie, contenant de la silice pyrogénée, présentant de bonnes propriétés cosmétiques et ne présentant pas les inconvénients ci-dessus.

La Demanderesse a découvert qu'une telle composition pouvait être obtenue en associant à la silice pyrogénée un épaississant particulier.

La présente invention a donc pour objet une composition comprenant de la silice pyrogénée, caractérisée par le fait qu'elle comprend également au moins un alkyléther de polysaccharide formé de motifs comportant au moins deux cycles osidiques différents, chaque motif comportant au moins un groupe hydroxyle substitué par une chaîne alkyle hydrocarbonée saturée, et une phase organique comprenant au moins un milieu solvant dudit alkyléther.

Grâce à l'association de la silice pyrogénée et de l'alkyléther de polysaccharide, la composition selon l'invention est transparente ou translucide et présente une viscosité élevée. En outre, la composition présente de bonnes propriétés cosmétiques : lors de l'application sur les matières kératiniques, la composition n'est pas collante ; elle est facile à appliquer et offre un confort pendant et après son application, sans laisser de sensation de tiraillement. En particulier, il est possible d'obtenir un bâton de rouge à lèvres doux à l'application et laissant sur les lèvres un film homogène et couvrant, confortable à porter, qui ne dessèche pas les lèvres. De plus, elle présente un aspect plus brillant que celle de l'art antérieur contenant de la silice.

Bien que l'invention soit particulièrement bien adaptée au domaine cosmétique, elle s'applique à tout domaine nécessitant d'obtenir des compositions épaisses voire des compositions solides et notamment dans les domaines agro-alimentaire, vétérinaire, dermatologique, pharmaceutique, du bois (stick de pigments et de cire pour la restauration de meubles).

Dans l'épaississant associé à la silice pyrogénée, on entend par « chaîne alkyle hydrocarbonée » une chaîne linéaire ou ramifiée, comportant de 1 à 24, de préférence de 1 à 10, mieux de 1 à 6 et plus spécialement de 1 à 3 atomes de carbone. En particulier, la chaîne alkyle est choisie parmi les chaînes saturées et notamment méthyle, éthyle, éthényle, n-propyle, propényle, isopropyle, n-butyle, isobutyle, tertiobutyle, n-pentyle. Ces alkyléthers peuvent être fabriqués comme décrits dans les documents EP-A-281 360, EP-A-708 114, EP-A-281360.

Selon un mode de réalisation préféré de l'invention, l'alkyléther de polysaccharide a un poids moléculaire moyen en poids supérieur à 100 000, et de préférence supérieur à 200 000. Ce poids moléculaire peut aller jusqu'à 1 million. Cet alkyléther peut comporter de un à six et mieux de deux à quatre groupes hydroxyle par motif, substitués par une chaîne alkyle hydrocarbonée saturée ou non.

Les cycles osidiques sont notamment choisis parmi le mannose, le galactose, le glucose, le furanose, le rhamnose, l'arabinose.

Selon un mode préféré de réalisation de l'invention, l'alkyléther de polysaccharide est un alkyléther d'une gomme et plus particulièrement d'une gomme globalement non ionique, c'est-à-dire comportant peu ou pas de groupe ionique. Comme gommes appropriées, on peut citer par exemple la gomme de guar dont le motif comprend un galactose et un mannose, la gomme de caroube dont le motif comprend un galactose et un mannose, la gomme de karaya qui est un mélange complexe de rhamnose, galactose et acide galacturonique, la gomme adragante qui est un mélange complexe d'arabinose, galactose et acide galacturonique.

Selon un mode de réalisation préféré de l'invention, l'alkyléther de polysaccharide est un dérivé de gomme de guar. Ainsi, avantageusement l'alkyléther est un galactomannane alkylé de chaîne alkyle en C₁ à C₆ et mieux en C₁ à C₃ et plus particulièrement le guar éthylé ayant un degré de substitution de 2 à 3 et notamment d'environ 2,5 à 2,8, tel que décrit dans les documents RD 95378007 (octobre 1995) et EP-A-708114. Cette gomme est en particulier celles vendues par la société Aqualon sous les noms N-HANCE-AG 200® et N-HANCE AG 50® .

La concentration en alkyléther dépend de la forme galénique, de la consistance recherchées pour la composition. En particulier le rapport en poids de la quantité de solvant et/ou d'huile sur la quantité d'épaississant est choisi par exemple dans la gamme allant de 5 à 1000. La composition selon l'invention peut contenir par exemple une quantité d'alkyléther de polysaccharide allant de 0,1 à 16 % en poids, par rapport au poids total de la phase organique (solvant et/ou huile) de la composition, et de préférence de 0,5 à 8 % en poids.

La silice pyrogénée susceptible d'être utilisée dans la composition selon l'invention peut se présenter sous forme de silice pyrogénée hydrophile ou de silice pyrogénée hydrophobe.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leurs surface. De telles silices hydrophiles sont par exemple commercialisées sous les dénominations "AEROSIL 130® ", "AEROSIL 200® ", "AEROSIL 255® ", "AEROSIL 300® ", "AEROSIL 380® " par la société Degussa, "CAB-O-SIL HS-5® ", "CAB-O-SIL EH-5® ", "CAB-O-SIL LM-130® ", "CAB-O-SIL MS-55® ", "CAB-O-SIL M-5® " par la société Cabot.

Il est possible de modifier chimiquement la surface de ladite silice, par réaction chimique générant une diminution du nombre de groupes silanol. On peut notamment substituer des groupes silanol par des groupements hydrophobes : on obtient alors une silice hydrophobe.

Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6^{ème} édition, 1995). Elles sont par exemple commercialisées sous les références "AEROSIL R812® " par la société Degussa, "CAB-O-SIL TS-530® " par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6^{ème} édition, 1995). Elles sont par exemple commercialisées sous les références "AEROSIL R972® ", "AEROSIL R974® " par la société Degussa, "CAB-O-SIL TS-610® ", "CAB-O-SIL TS-720® " par la société Cabot.

Selon l'invention, on utilise de préférence des silices hydrophobes.

La silice pyrogénée présente de préférence une taille de particules pouvant être nanométrique à micrométrique, par exemple allant d'environ de 5 à 200 nm.

La composition selon l'invention peut comprendre de la silice pyrogénée en une quantité allant de 0,5 % à 25 % en poids, par rapport au poids total de la phase organique de la composition, de préférence de 1 % à 20 % en poids, et mieux de 1 % à 10 % en poids.

Selon l'invention, le milieu solvant de l'alkyléther de polysaccharide présent dans la composition peut être un solvant organique ou une huile. En d'autres termes, l'alkyléther de polysaccharide est un épaississant des solvants organiques et des huiles. Par huiles, on entend toute matière grasse liquide à température ambiante.

Le solvant organique peut être, par exemple, choisi parmi :
- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les composés cycliques aromatiques liquides à température ambiante tels que le toluène et le xylène ;
- les aldéhydes liquides à température ambiante tels que le benzaldéhyde, l'acétaldéhyde ;

Ces solvants conviennent plus particulièrement pour le maquillage ou le démaquillage des ongles : la composition constitue alors un vernis à ongles, un produit de soin des ongles ou un dissolvant de vernis à ongles.

Parmi les, huiles utilisables comme milieu solvant de l'alkyléther de polysaccharide selon l'invention, on peut citer par exemple :
- les huiles d'origine végétale comme les triglycérides liquides, par exemple les huiles de tournesol, de maïs, de soja, de jojoba, de courge, de pépins de raisin, de sésame, de noisette, d'abricot, de macadamia, de ricin, les triglycérides des acides caprylique/caprique comme ceux vendus par la société STEARINERIES DUBOIS ou ceux vendus sous les dénominations MIGLYOL 810, 812 et 818 par la société DYNAMIT NOBEL.
- les huiles d'origine animale telle que la lanoline,
- les huiles d'origine minérale,
- les huiles de synthèse comme les alcools gras tels que octyl-2-dodécanol; les esters et en particulier les esters d'acides gras, et notamment les esters ayant un nombre total d'atomes de carbone choisi entre 12 et 80 et mieux entre 16 et 50; les silicones phénylées, et notamment les phényl triméthicones, les diphényl diméthicones, les polyméthylphényl siloxanes.

L'homme du métier sait, par ses connaissances, déterminer par de simples essais de routine les huiles solubilisant l'alkyléther de polysaccharide.

Des huiles complémentaires, non solvant de l'alkyléther de polysaccharide, peuvent en outre être ajoutées dans la composition. Comme huile complémentaire, on peut notamment citer les résines et les gommes de silicone liquides à température ambiante, les huiles hydrocarbonées partiellement fluorées, les huiles perfluorées, les huiles siliconées exemptes de groupements aromatiques telles que les polysiloxanes linéaires ou ramifiés comme les polydiméthylpolysiloxanes, les polyéthylméthylpolysiloxanes, polyalkylméthylsiloxanes et les polysiloxanes cycliques tels que octaméthylcyclotétrasiloxane, décaméthylcyclopentasiloxane ou leurs mélanges ; les huiles de silicones fluorées ; les polysiloxanes fonctionnalisés par une ou plusieurs fonctions hydroxyles et/ou un ou plusieurs groupements polyéthers tels que les diméthicones copolyols ; les hydrocarbures linéaires ou ramifiés, comme l'huile de vaseline, l'isohexadécane, l'isododécane.

Les solvants de l'alkyléther de polysaccharide (solvant organique ou huile) peuvent être présents à raison de 59 à 99,4 % en poids, par rapport au poids total de la phase organique de la composition, et mieux de 72 % à 98,5 %. Les huiles complémentaires peuvent être ajoutées dans la composition en une quantité pouvant aller de 0 % à 75 % en poids, par rapport au poids total de la phase organique, et mieux de 0 % à 50 % en poids.

Il est en outre possible d'incorporer dans la composition selon l'invention une phase aqueuse, notamment en une quantité allant de 0 % à 95 % en poids par rapport au poids total de la composition, et mieux de 3 à 85 %.

La composition selon l'invention peut de plus comprendre tous les ingrédients classiquement utilisés dans les domaines concernés et plus spécialement dans les domaines cosmétique et dermatologique. Ces ingrédients sont en particuliers choisis parmi les conservateurs, les vitamines, les épaississants de phase aqueuse ou grasse différents de celui de l'invention, les parfums, les tensioactifs, les antioxydants, les charges, les pigments, les cires, les actifs cosmétiques ou dermatologiques liposolubles ou hydrosolubles, et leurs mélanges. La composition selon l'invention peut contenir également des vésicules lipidiques de type ionique et/ou non ionique. Les quantités de ces différents ingrédients sont celles classiquement utilisées dans les domaines concernés et par exemple de 0,01 % à 20 % du poids total de la composition. La nature de ces ingrédients et leur proportion doit être compatibles avec l'obtention de compositions stables épaissies selon l'invention.

La composition de l'invention peut se présenter sous toutes les formes galéniques normalement utilisées pour une application topique ou orale, et notamment sous forme d'un gel, d'une émulsion, d'une dispersion, de système multiphases et notamment biphasique. Cette composition peut avoir l'aspect d'un lait, d'une crème, d'une pâte, d'un solide coulé ou moulé et notamment d'un stick.

La composition selon l'invention peut être avantageusement utilisée pour le traitement, le maquillage (vernis à ongles, eye-liner, mascara, fond de teint, anticernes, fard à paupières ou à joues, rouge à lèvres), ou le soin des matières kératiniques et/ou des muqueuses selon la nature des actifs utilisés.

L'invention se rapporte également à l'utilisation d'une silice pyrogénée et d'un alkyléther de polysaccahride tels que définis précédemment comme agent épaississant d'une composition comprenant une phase organique comprenant au moins un milieu solvant dudit alkyléther.

L'invention se rapporte aussi à un procédé de traitement cosmétique, de maquillage ou de démaquillage des matières kératiniques et/ou des muqueuses consistant à appliquer sur les matières kératiniques et/ou les muqueuses une composition telle que décrite précédemment.

Les exemples ci-après sont donnés à titre d'illustration et sans caractère limitatif.

### Exemple 1 :

On a préparé un rouge à lèvres selon l'invention et un rouge à lèvres comparatif ne comprenant que de la silice pyrogénée comme épaississant. Les compositions étaient les suivantes, les quantités étant exprimées en gramme :

| **Composition** | **invention** | **comparative** |
|---|---|---|
| Huile de sésame | 31,85 | 32,2 |
| Triheptanoate de glycéryle | 31,85 | 32,2 |
| Silice pyrogénée hydrophobe (1) | 5,6 | 5,6 |
| guar éthylé de degré de substitution d'environ 2,5(2) | 0,7 | - |
| Lanoline liquide | 12 | 12 |
| Cire de polyéthylène | 10 | 10 |
| Pigment | 8 | 8 |

| | | |
|---|---|---|
| (1) vendue sous la dénomination AEROSIL R972® par Degussa | | |
| (2) vendu sous la dénomination N-HANCE AG 200® par Aqualon | | |

On a ajouté l'éthylguar dans le mélange d'huiles chauffé à 90-95 °C pour former le gel puis on a dispersé la silice pyrogénée dans le gel et on a ajouté les pigments. Le mélange a été broyé par trois passages à la broyeuse tricylindre. Puis on a incorporé la cire de polyéthylène et chauffé le mélange à 100-105 °C. On a coulé le mélange à la température de 98-100 °C dans un moule, puis on a laissé refroidir.

Après démoulage, on a évalué les propriétés cosmétiques des deux produits.

On a constaté que le rouge à lèvres selon l'invention laisse sur les lèvres un film plus couvrant, plus homogène, plus confortable à porter, moins poudreux que celui obtenu avec le produit comparatif. En outre, le rouge selon l'invention dessèche moins les lèvres.

### Exemple 2 :

On a préparé un rouge à lèvres pâteux ayant la composition suivante :
- Silice pyrogénée hydrophobe (1) 7,64 g
- guar éthylé de degré de substitution d' environ 2,5 (2) 0,59 g
- Phényltriméthicone vendue sous la dénomination DC 556® par la société Dow Corning 89,77 g
- pigments 2 g
   (1) vendue sous la dénomination AEROSIL R972® par Degussa
   (2) vendu sous la dénomination N-HANCE AG 200® par Aqualon

On a préparé le mélange d'huile, de silice et de guar éthylé selon le mode opératoire donné à l'exemple 1. On a ajouté ensuite les pigments puis broyé le mélange par trois passages à la broyeuse tricylindre. On a obtenu une pâte qui s'applique facilement sur les lèvres et qui laisse un film confortable à porter sans dessécher les lèvres.

### Exemple 3 :

On a préparé, selon le mode opératoire de l'exemple 2, un rouge à lèvres pâteux ayant la composition suivante :
- Silice pyrogénée hydrophobe (1) 7,52 g
- guar éthylé de degré de substitution d' environ 2,5 (2) 0,94 g
- Triheptanoate de glycéryle 85,54 g
- nacres 6 g
   (1) vendue sous la dénomination AEROSIL R972® par Degussa
   (2) vendu sous la dénomination N-HANCE AG 200® par Aqualon

On a obtenu une pâte qui s'applique confortablement sur les lèvres, présente un bon pouvoir couvrant et le film déposé ne procure pas de sensation de tiraillement.

### Exemple 4 :

On a préparé, selon le mode opératoire de l'exemple 2, un rouge à lèvres pâteux ayant la composition suivante :
- Silice pyrogénée hydrophobe (1) 7,52 g
- guar éthylé de degré de substitution d'environ 2,5 (2) 0,94 g
- huile de sésame 85,54 g
- nacres 6 g
   (1) vendue sous la dénomination AEROSIL R972® par Degussa
   (2) vendu sous la dénomination N-HANCE AG 200® par Aqualon

On a obtenu une pâte qui s'applique facilement sur les lèvres et laisse sur celles-ci un film homogène, confortable à porter.

### Exemple 5 :

- Silice pyrogénée hydrophobe (3) 10 g
- guar éthylé de degré de substitution d' environ 2,5 (2) 2 g
- octyl-2 dodécanol 88 g
   (3) vendue sous la dénomination AEROSIL R974® par Degussa

On a obtenu un gel transparent ayant une viscosité de 7,3 Pa. S (mesurée au Contraves TBS, à 25 °C, équipé d'un mobile n° 4 tournant à la fréquence de 50 Hz). Ce gel peut servir de base à toute composition cosmétique ou dermatologique.

### Exemple 6 :

- Silice pyrogénée hydrophobe (3) 10 g
- guar éthylé de degré de substitution d'environ 2,5 (2) 2 g
- huile de ricin 88 g
   (3) vendue sous la dénomination AEROSIL R974® par Degussa

On a obtenu un gel transparent ayant une viscosité de 57,5 Pa. S (mesurée au Contraves TBS, à 25 °C, équipé d'un mobile n° 5 tournant à la fréquence de 50 Hz). Ce gel peut servir de base à toute composition cosmétique ou dermatologique.

## Revendications

1. Composition comprenant de la silice pyrogénée, caractérisée par le fait qu'elle comprend également au moins un alkyléther de polysaccharide formé de motifs comportant au moins deux cycles osidiques différents, chaque motif comportant au moins un groupe hydroxyle substitué par une chaîne alkyle hydrocarbonée saturée, et une phase organique comprenant au moins un milieu solvant dudit alkyléther.

2. Composition selon la revendication 1, caractérisée par le fait que deux à quatre groupes hydroxyle par motif sont substitués par une chaîne alkyle hydrocarbonée saturée.

3. Composition selon l'une quelconque des revendications précédentes, caractérisée par le fait que la chaîne alkyle hydrocarbonée saturée comporte de 1 à 24 atomes de carbone.

4. Composition selon l'une quelconque des revendications précédentes, caractérisée par le fait que la chaîne alkyle hydrocarbonée saturée comporte de 2 à 10 atomes de carbone.

5. Composition selon l'une quelconque des revendications précédentes, caractérisée par le fait que la chaîne alkyle est choisie dans le groupe formé par les radicaux méthyle, éthyle, n-propyle, isopropyle, n-butyle, isobutyle, tertiobutyle.

6. Composition selon l'une quelconque des revendications précédentes, caractérisée par le fait que les cycles osidiques sont choisis dans le groupe formé par le mannose, le galactose, le glucose, le furanose, le rhamnose, l'arabinose.

7. Composition selon l'une quelconque des revendications précédentes, caractérisée par le fait que l'alkyléther de polysaccharide est un alkyléther d'une gomme choisie parmi la gomme de guar, la gomme de caroube, la gomme de karaya, la gomme adragante et leurs mélanges.

8. Composition selon l'une quelconque des revendications précédentes, caractérisée par le fait que l'alkyéther est un galactomannane alkylé de chaîne alkyle en C₁ à C₆ et mieux en C₁ à C₃.

9. Composition selon l'une quelconque des revendications précédentes, caractérisée par le fait que l'alkyléther de polysaccharide est de la gomme de guar à chaîne éthyle avec un degré de substitution de 2 à 3.

10. Composition selon l'une quelconque des revendications précédentes, caractérisée par le fait que l'alkyléther de polysaccharide a un poids moléculaire moyen en poids supérieur à 200 000.

11. Composition selon l'une quelconque des revendications précédentes, caractérisée par le fait que l'alkyléther de polysaccharide est présent en une quantité telle que le rapport (en poids) de la quantité de solvant et/ou d'huile sur la quantité d'alkyléther de polysaccharide est choisi dans la gamme allant de 5 à 1000.

12. Composition selon l'une quelconque des revendications précédentes, caractérisée par le fait que l'alkyléther de polysaccharide est présent en une quantité allant de 0,1 à 16 % du poids total de la phase organique et mieux de 0,5 à 8 % du poids total de la phase organique de la composition.

13. Composition selon l'une quelconque des revendications précédentes, caractérisée par le fait que la silice pyrogénée est choisi parmi les silices pyrogénées hydrophobes.

14. Composition selon l'une quelconque des revendications précédentes, caractérisée par le fait que la silice pyrogénée est présente en une quantité allant de 0,5 % à 25 % en poids, de préférence de 1 à 20 % en poids, par rapport au poids total de la phase organique de la composition.

15. Composition selon l'une quelconque des revendications précédentes, caractérisée par le fait que le milieu solvant dudit alkyléther est un solvant organique et/ou une huile.

16. Composition selon l'une des revendications précédentes, caractérisée par le qu'elle comprend au moins une huile complémentaire non solvante de l'alkyléther de polysaccharide.

17. Composition selon l'une quelconque des revendications précédentes, caractérisée par le fait qu'elle comprend en outre au moins un ingrédient choisi dans le groupe formé par les conservateurs, les vitamines, les parfums, les tensioactifs, les antioxydants, les charges, les pigments, les cires, et leurs mélanges.

18. Composition selon l'une quelconque des revendications précédentes, caractérisée par le fait qu'elle se présente sous la forme d'un gel, d'une émulsion, d'une dispersion, d'un système multiphase.

19. Composition, selon l'une quelconque des revendications précédentes, caractérisée par le fait qu'elle se présente sous la forme d'un rouge à lèvres.

20. Utilisation d'une silice pyrogénée associée à un alkyléther de polysaccharide formé de motifs comportant au moins deux cycles osidiques différents, chaque motif comportant au moins un groupe hydroxyle substitué par une chaîne alkyle hydrocarbonée saturée comme agent épaississant d'une composition comprenant une phase organique comprenant au moins un milieu solvant dudit alkyléther.

21. Utilisation selon la revendication 20, caractérisée par le fait que l'alkyléther de polysaccharide est un alkyléther d'une gomme choisie parmi la gomme de guar, la gomme de caroube, la gomme de karaya, la gomme adragante et leurs mélanges.

22. Procédé de traitement cosmétique des matières kératiniques et/ou des muqueuses, caractérisé par le fait que l'on applique sur les matières kératiniques et/ou sur les muqueuse une composition selon l'une quelconque des revendications 1 à 19.

23. Procédé de maquillage des matières kératiniques et/ou des muqueuses, caractérisé par le fait que l'on applique sur les matières kératiniques et/ou sur les muqueuse une composition selon l'une quelconque des revendications 1 à 19.

## Claims

1. Composition comprising fused silica, characterized in that it also comprises at least one polysaccharide alkyl ether formed of units containing at least two different saccharide rings, each unit containing at least one hydroxyl group substituted with a saturated hydrocarbon-based alkyl chain, and an organic phase comprising at least one medium which is a solvent for the said alkyl ether.

2. Composition according to Claim 1, characterized in that two to four hydroxyl groups per unit are substituted with a saturated hydrocarbon-based alkyl chain.

3. Composition according to either of the preceding claims, characterized in that the saturated hydrocarbon-based alkyl chain contains from 1 to 24 carbon atoms.

4. Composition according to any one of the preceding claims, characterized in that the saturated hydrocarbon-based alkyl chain contains from 2 to 10 carbon atoms.

5. Composition according to any one of the preceding claims, characterized in that the alkyl chain is chosen from the group formed by the methyl, ethyl, n-propyl, isopropyl, n-butyl, isobutyl and tert-butyl radicals.

6. Composition according to any one of the preceding claims, characterized in that the saccharide rings are chosen from the group formed by mannose, galactose, glucose, furanose, rhamnose and arabinose.

7. Composition according to any one of the preceding claims, characterized in that the polysaccharide alkyl ether is an alkyl ether of a gum chosen from guar gum, carob gum, karaya gum and gum tragacanth, and mixtures thereof.

8. Composition according to any one of the preceding claims, characterized in that the alkyl ether is an alkyl galactomannan with a C₁ to C₆, and better still C₁ to C₃, alkyl chain.

9. Composition according to any one of the preceding claims, characterized in that the polysaccharide alkyl ether is guar gum containing an ethyl chain with a degree of substitution of from 2 to 3.

10. Composition according to any one of the preceding claims, characterized in that the polysaccharide alkyl ether has a weight-average molecular weight of greater than 200,000.

11. Composition according to any one of the preceding claims, characterized in that the polysaccharide alkyl ether is present in an amount such that the ratio (by weight) of the amount of solvent and/or oil to the amount of polysaccharide alkyl ether is chosen in the range from 5 to 1000.

12. Composition according to any one of the preceding claims, characterized in that the polysaccharide alkyl ether is present in an amount ranging from 0.1 to 16% of the total weight of the organic phase, and better still from 0.5 to 8% of the total weight of the organic phase of the composition.

13. Composition according to any one of the preceding claims, characterized in that the fused silica is chosen from hydrophobic fused silicas.

14. Composition according to any one of the preceding claims, characterized in that the fused silica is present in an amount ranging from 0.5% to 25% by weight, preferably from 1 to 20% by weight, relative to the total weight of the organic phase of the composition.

15. Composition according to any one of the preceding claims, characterized in that the medium which is a solvent for the said alkyl ether is an organic solvent and/or an oil.

16. Composition according to one of the preceding claims, characterized in that it comprises at least one complementary oil which is not a solvent for the polysaccharide alkyl ether.

17. Composition according to any one of the preceding claims, characterized in that it also comprises at least one ingredient chosen from the group formed by preserving agents, vitamins, fragrances, surfactants, antioxidants, fillers, pigments and waxes, and mixtures thereof.

18. Composition according to any one of the preceding claims, characterized in that it is in the form of a gel, an emulsion, a dispersion or a multiphase system.

19. Composition according to any one of the preceding claims, characterized in that it is in the form of a lipstick.

20. Use of a fused silica combined with a polysaccharide alkyl ether formed of units containing at least two different saccharide rings, each unit containing at least one hydroxyl group substituted with a saturated hydrocarbon-based alkyl chain, as a thickener for a composition comprising an organic phase comprising at least one medium which is a solvent for the said alkyl ether.

21. Use according to Claim 20, characterized in that the polysaccharide alkyl ether is an alkyl ether of a gum chosen from guar gum, carob gum, karaya gum and gum tragacanth, and mixtures thereof.

22. Cosmetic process for treating keratinous material and/or mucous membranes, characterized in that a composition according to any one of Claims 1 to 19 is applied to the keratinous material and/or to the mucous membranes.

23. Process for making up keratinous material and/or mucous membranes, characterized in that a composition according to any one of Claims 1 to 19 is applied to the keratinous material and/or to the mucous membranes.

## Patentansprüche

1. Zusammensetzung, die pyrogene Kieselsäure enthält, dadurch gekennzeichnet, daß sie mindestens einen Polysaccharidalkylether, der aus Einheiten gebildet ist, die mindestens zwei verschiedene Zuckerringe aufweisen, wobei jede Einheit mindestens eine Hydroxygruppe enthält, die mit einer gesättigten Alkylgruppe (Kohlenwasserstoffgruppe) substituiert ist, und eine organische Phase enthält, die mindestens ein Lösungsmittelmedium für den Alkylether enthält.

2. Zusammensetzung nach Anspruch 1, dadurch gekennzeichnet, daß pro Einheit 2 bis 4 Hydroxygruppen mit einer gesättigten Alkylgruppe substituiert sind.

3. Zusammensetzung nach einem der vorhergehenden Ansprüche, dadurch gekennzeichnet, daß die gesättigte Alkylgruppe 1 bis 24 Kohlenstoffatome aufweist.

4. Zusammensetzung nach einem der vorhergehenden Ansprüche, dadurch gekennzeichnet, daß die gesättigte Alkylgruppe 2 bis 10 Kohlenstoffatome aufweist.

5. Zusammensetzung nach einem der vorhergehenden Ansprüche, dadurch gekennzeichnet, daß die Alkylgruppe unter Methyl, Ethyl, n-Propyl, Isopropyl, n-Butyl, Isobutyl und tert.-Butyl ausgewählt ist.

6. Zusammensetzung nach einem der vorhergehenden Ansprüche, dadurch gekennzeichnet, daß die Zuckerringe unter Mannose, Galactose, Glucose, Furanose, Rhamnose und Arabinose ausgewählt sind.

7. Zusammensetzung nach einem der vorhergehenden Ansprüche, dadurch gekennzeichnet, daß der Polysaccharidalkylether ein Alkylether eines Gummis ist, das unter Guargummi, Johannisbrotkernmehl, Karaya-Gummi und Tragant und deren Gemischen ausgewählt ist.

8. Zusammensetzung nach einem der vorhergehenden Ansprüche, dadurch gekennzeichnet, daß es sich bei dem Alkylether um ein alkyliertes Galactomannan mit einer C₁₋₆-Alkylgruppe und besser noch einer C₁₋₃-Alkylgruppe handelt.

9. Zusammensetzung nach einem der vorhergehenden Ansprüche, dadurch gekennzeichnet, daß der Polysaccharidalkylether ein Guargummi mit Ethylgruppe ist, das einen Substitutionsgrad von 2 bis 3 aufweist.

10. Zusammensetzung nach einem der vorhergehenden Ansprüche, dadurch gekennzeichnet, daß der Polysaccharidalkylether ein Gewichtsmittel des Molekulargewichts über 200 000 aufweist.

11. Zusammensetzung nach einem der vorhergehenden Ansprüche, dadurch gekennzeichnet, daß der Polysaccharidalkylether in einem solchen Mengenanteil vorliegt, daß das Verhältnis (auf das Gewicht bezogen) der Menge des Lösungsmittel und/oder des Öls und der Menge des Polysaccharidalkylethers im Bereich von 5 bis 1000 liegt.

12. Zusammensetzung nach einem der vorhergehenden Ansprüche, dadurch gekennzeichnet, daß der Polysaccharidalkylether in einem Mengenanteil von 0,1 bis 16 % des Gesamtgewichts der organischen Phase und besser noch 0,5 bis 8 % des Gesamtgewichts der organischen Phase der Zusammensetzung vorliegt.

13. Zusammensetzung nach einem der vorhergehenden Ansprüche, dadurch gekennzeichnet, daß die pyrogene Kieselsäure unter den hydrophoben pyrogenen Kieselsäuren ausgewählt ist.

14. Zusammensetzung nach einem der vorhergehenden Ansprüche, dadurch gekennzeichnet, daß die pyrogene Kieselsäure in einem Mengenanteil von 0,5 bis 25 Gew.-% und vorzugsweise 1 bis 20 Gew.-%, bezogen auf das Gesamtgewicht der organischen Phase der Zusammensetzung, vorliegt.

15. Zusammensetzung nach einem der vorhergehenden Ansprüche, dadurch gekennzeichnet, daß das Lösungsmittelmedium für den Alkylether ein organisches Lösungsmittel und/oder ein Öl ist.

16. Zusammensetzung nach einem der vorhergehenden Ansprüche, dadurch gekennzeichnet, daß sie mindestens ein zusätzliches Öl enthält, das kein Lösungsmittel für den Polysaccharidalkylether darstellt.

17. Zusammensetzung nach einem der vorhergehenden Ansprüche, dadurch gekennzeichnet, daß sie mindestens einen Bestandteil enthält, der unter den Konservierungsmitteln, Vitaminen, Parfüms, grenzflächenaktiven Stoffen, Antioxidantien, Füllstoffen, Pigmenten, Wachsen und deren Gemischen ausgewählt ist.

18. Zusammensetzung nach einem der vorhergehenden Ansprüche, dadurch gekennzeichnet, daß sie als Gel, Emulsion, Dispersion oder Multiphasensystem vorliegt.

19. Zusammensetzung nach einem der vorhergehenden Ansprüche, dadurch gekennzeichnet, daß sie als Lippenstift vorliegt.

20. Verwendung einer pyrogenen Kieselsäure in Kombination mit einem Polysaccharidalkylether, der aus Einheiten gebildet ist, die mindestens zwei verschiedene Zuckerringe aufweisen, wobei jede Einheit mindestens eine Hydroxygruppe enthält, die mit einer gesättigten Alkylgruppe substituiert ist, als Verdickungsmittel in einer Zusammensetzung, die eine organische Phase enthält, welche mindestens ein Lösungsmittelmedium für den Alkylether aufweist.

21. Verwendung nach Anspruch 20, dadurch gekennzeichnet, daß der Polysaccharidalkylether ein Alkylether eines Gummis ist, das unter Guargummi, Johannisbrotkernmehl, Karaya-Gummi, Tragant oder deren Gemischen ausgewählt ist.

22. Verfahren zur kosmetischen Behandlung von Keratinsubstanzen und/oder Schleimhäuten, dadurch gekennzeichnet, daß auf die Keratinsubstanzen und/oder die Schleimhäute einer Zusammensetzung nach einem der Ansprüche 1 bis 19 aufgebracht wird.

23. Verfahren zum Schminken von Keratinsubstanzen und/oder Schleimhäuten, dadurch gekennzeichnet, daß auf die Keratinsubstanzen und/oder die Schleimhäute eine Zusammensetzung nach einem der Ansprüche 1 bis 19 aufgebracht wird.
